# EUROPEAN PATENT APPLICATION

(11) **EP 3 409 599 A1**
(43) Date of publication of application: **05.12.2018**
(21) Application number: 18020237.6
(22) Date of filing: 28.05.2018
(51) Int. Cl.: B65B 11/50, A61M 25/00

(54) **SYSTEM FOR ASEPTIC PACKAGING**

(30) Priority: 31.05.2017 IT 201700058894
(71) Applicant: Persico S.p.A., 24027 Nembro (BG) (IT)
(72) Inventor: Zaccaria, Antonio, 24027 Nembro (BG) (IT); Ollari, Ermes, 24027 Nembro (BG) (IT); Ortenzi, Carlo, 24027 Nembro (BG) (IT)
(74) Representative: Tirloni, Bartolomeo

(57) **Abstract**

A system for aseptic packaging (1) is described, regarding casings (E) containing functional objects (C) in contact with substances (L), by positioning and finishing of the casings (E) and of the functional objects (C) and by dispensing of the substances (L), comprising means for performing positioning operations of the open casings (E), positioning and finishing of the functional objects (C) inside the open casings (E), calibrated dispensing of the substances (L) inside the open casings (E), and closing and finishing of the casings (E).

A positioning and calibrated dispensing device (10) allows the positioning of the functional objects (C) inside the open casings (E) and the calibrated dispensing of the substances (L) inside the open casings (E).

## Description

### Technical Field

The present invention relates to a system for aseptic packaging.

In general, the present invention refers to bonding or sealing pre-formed parts, for example, welding of plastic materials, machines used for the welding and cutting or for bonding and shearing, in which the cut is made in the area to be joined, next to the area to be joined in the area of joining or near the junction area using a single unit having both a cutting tool and a welding tool.

Such machines can relate to the packaging of catheters, probes, perforated containers, bags of blood, medical bags.

In particular, the present invention relates to the packaging of catheters lubricated.

A catheter lubricated consists of a plastic tube, possibly connected to a plastic connector, packaged within a hermetic protective bag containing a lubricant, for example, glycerin, to facilitate the use of the catheter.

The choice of the welding parameters, for example, heat welding or high frequency or of another type, between the catheter and hermetic bag depends on the material of the catheter and of the thin film used for the hermetic bag.

Intermittent bladder catheters, ISO 09.24.06, self-lubricating catheters, SIVA 09.24.06.S01, are known.

In addition to the catheter, i.e. the probe which is introduced into the body of the patient, the hermetic bag also contains a certain amount of lubricant, for example, from a few grams to about 50 grams. The catheter may join a collecting bag, for example, of liquids or terminate with a fitting, which in turn is connected to other elements.

The packaging steps of catheters lubricated envisage: positioning of the catheter tube, i.e. the tube possibly connected to the connector; welding of the catheter joined to the film, usually the film formed by a pair of layers; welding of the contour of the film leaving an opening for the introduction of the lubricant; introduction of the lubricant; welding of the point of introduction of the lubricant and of other flaps of the bag to make it completely hermetic.

### Background Art

The state of the art is represented by patent EP 1 737 648 B1 concerning a combined machine and a process for manufacturing bags by means of a plastic film. The combined machine comprises one or more dispensers of film, one or more dispensers of tubular elements, a combined cutting/welding tool for fixing to the bags of plastic element, another welding jig for the welding of the tubular elements and a transfer station to allow a further processing of plastic bags. The simplified production process allows creating a combined compact machine grouping in a single device the dispensers of film, the dispensers of tubular elements, the combined cutting/welding tool, and the welding transfer station.

Moreover, it is known the patent application US 2005/0137619 A1 regarding a method for providing a medical flask comprising the steps of providing a polymeric tube having two ends, removing the tube axially, expansion of the tube radially leaving the ends of the tube free to move axially in response to radial expansion. In particular, the step of expansion of the tube comprises the positioning of the tube in a mold and at the same time the heating and the pressurizing of the tube.

Thus, it is also known the US patent 7,770,726 B2 concerning a sheet wound on a catheter to form a casing. The catheter extends generally longitudinally inside the casing and the sheet extends from a point beyond the proximal end to a point beyond the distal end of the catheter. The end edges, proximal and distal side of the sheet are joined by means of a gasket to define a sealed hollow. The sheet has a tear strip adapted to cause the opening of the casing along a pre-formed opening line. A barrier impermeable to liquid and gas permeable divides the cavity sealed in a first compartment containing the catheter and in a second compartment containing a substance wetted with an aqueous liquid. Said barrier is advantageously heat sealed to the internal surface of the enclosure to cover the wet substance. According to an automated method, the sheet is unwound from a roll toward a reception point of the catheter. The catheter advances on top of the sheet until such reception point, while the tear strip is positioned on the sheet wound in shape of a U. The automatic method provides for the sealing of the sheet so as to form the separate cavities, sealed and subsequently cut so as to form a series of separate casings, each casing containing its own catheter.

Despite these efforts, there remains a need to improve the process of aseptic packaging of catheters lubricated by the reduction in the cycle times and the simplification of the construction.

### Summary of invention

The object of the present invention is solving the above prior art problems by integrating more steps of this method in the same operating unit in order to reduce the number of operating units necessary generally.

A further object is to increase the simultaneity of the operations in a continuous cycle of packaging, in order to eliminate the time of no-use of operating units.

The above and other objects and advantages of the invention, as will appear from the following description are achieved with a system of aseptic packaging as described in the claims.

Preferred embodiments and non-trivial variations of the present invention are claimed in the dependent claims.

It is to be understood that all the claims form an integral part of the present description.

It will be immediately obvious to the skilled people in the art that could be performed to what has been described numerous variations and modifications (for example related to shape, sizes, arrangements and parts with equivalent functionality) without deviating from the scope of protection of the invention as appears from the enclosed claims.

### Brief description of drawings

The present invention will be better described by some preferred embodiments thereof, given as a non-limiting example, with reference to the enclosed drawings, in which:
FIG. 1 shows a front view of an embodiment of the system for aseptic packaging, according to the present invention;
FIG. 2 shows a plan view of an embodiment of the system of the preceding figure;
FIG. 3 shows a plan view of a finished package concerning an embodiment of the system for aseptic packaging, according to the present invention;
FIG. 4 shows a front view of a component belonging to an embodiment of the system for aseptic packaging, according to the present invention;
FIG. 5 shows a plan view of the component of the preceding figure;
FIG. 6 shows an enlarged detail VI of FIG. 4; and
FIG. 7 shows an axonometric view of the component of FIGS. 4, 5, 6.

### Description of embodiments

With reference to the figures, it is possible to note that a system for aseptic packages 1 relates to casings E containing functional objects C in contact with substances L, by positioning and finishing the casings E and the functional objects C and by dispensing of the substances L.

In particular, the system 1 comprises devices for performing operations of: positioning the open casings E; positioning and finishing the functional objects C inside the open casings E; calibrated dispensing the substances L inside the open casings E; and closing and finishing the casings E.

Advantageously, the system 1 comprises a device for positioning and calibrated dispensing 10 to allow the positioning of the functional objects C inside the open casings E and the calibrated dispensing of the substances L inside the open casings E.

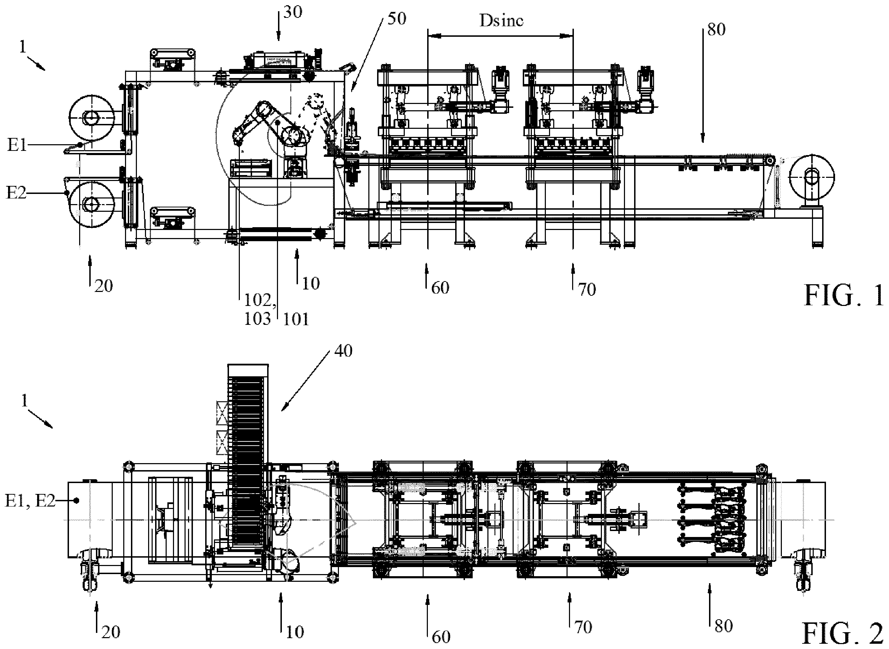

With reference to FIGS. 1, 2, the positioning of the open casings E relates to a first film layer E1 superimposed and facing a second film layer E2, to allow the positioning of the functional objects C between the first and the second layer of film E1, E2 and the calibrated dispensing of the substances L in proximity to the functional objects C, through the positioning and calibrated dispensing device 10.

The positioning and calibrated dispensing device 10 comprises a robotic arm 101 connected to gripping means 102 and to intercepting means of substances 103.

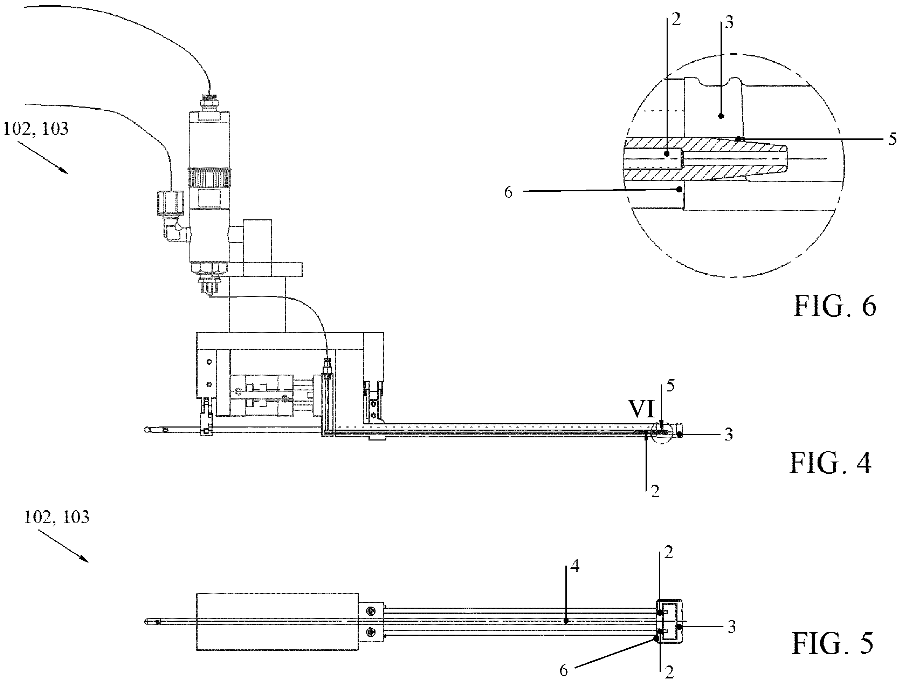

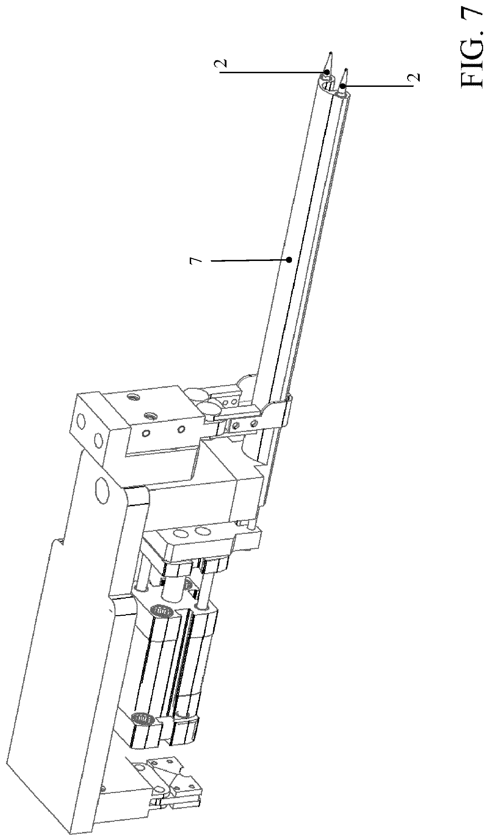

With reference to FIGS. 4 to 7, the gripping means 102 comprise at least one resilient tubular element 2 shaped so as to be able to join in at least one point of the functional objects C to allow the positioning of the functional objects C.

In addition, the intercepting means of substances 103 are connected to said at least one resilient tubular element 2, to enable the dispensing of the substances L.

With reference to FIGS. 1, 2, in-feeding means 20 of the first layer of film E1 and the second layer of film E2 are combined with a set of transmission, retraction, and accumulation/release elements to allow to configure the first layer of film E1 superimposed over and facing the second film layer E2.

Possible printing means 30 allow at least one of the layers of film E1, E2 to be graphically impressed.

In-feeding means 40 supply the functional objects C.

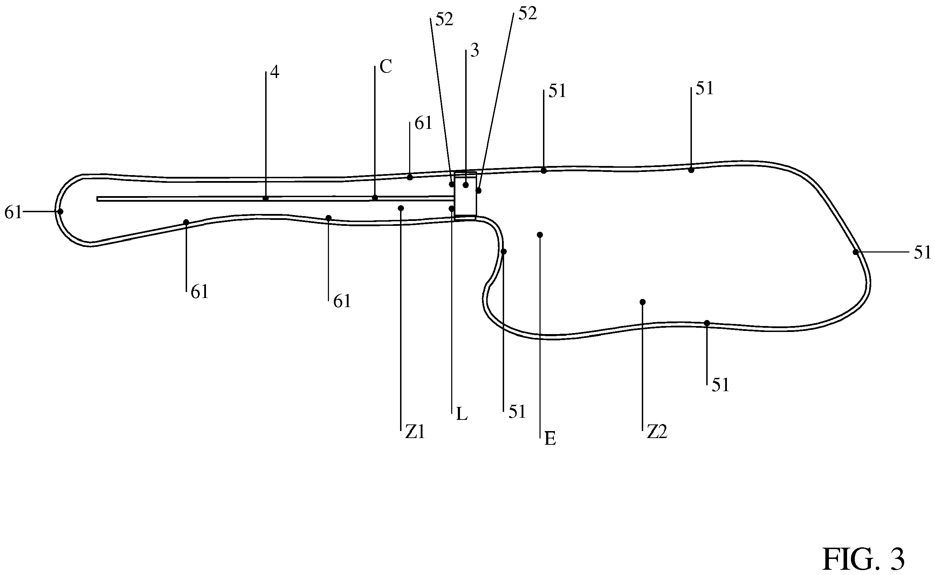

With reference to FIGS. 1 to 3, a first joining and welding device 50 allows to form the open casings E, by joining and welding of first edges 51, in addition to fix the functional objects C inside the open casing E, by joining and welding of second edges 52, creating two separate and distinct zones Z1, Z2, inside the casings E.

A second joining and welding device 60 allows to form the closed casings E, by joining and welding of third edges 61.

A hollow cutting device 70 allows the outer edging of the casings E.

Final evacuation means 80 serve to remove the finished package.

The two first and second devices for joining and welding, 50, 60, respectively are placed each other at a distance Dsinc to be able to operate simultaneously on casings E opened and closed respectively by providing a continuous cycle of packaging.

According to a preferred form of use of the packaging system 1, the casings E are formed of thin films of polyvinyl chloride and the functional objects C consist of catheters lubricated by the substances L, as glycerin.

In particular, the functional objects C consisting of catheters lubricated comprise a junction element 3 of plastic, positioned and welded hermetically so as to be able to separate the two zones Z1, Z2, inside the casings E.

Optionally, a tube 4 is connected to the junction element 3.

The junction element 3 comprises calibrated holes 5 and a mechanical stop 6 respectively, to allow connection and separation with respect to the gripping means 102, by means of the resilient tubular element 2 which is slidable with respect to a bracket 7.

A packaging process, with the system for aseptic packaging 1 comprises the following steps:
a1) by means of the in-feeding means 20, in-feeding of the first layer of film E1 and of the second layer of film E2;
a2) by means of the in-feeding means 40, in-feeding of the functional objects C;
b) by means of the printing media 30, optional graphics printing on at least one of the layers of film E1, E2;
c) by means of the device for positioning and calibrated dispensing 10, positioning of the functional objects C between the first and the second layer of film E1, E2;
d1) by means of the first joining and welding device 50, joining and welding of the first edges 51, so as to form the open casings E;
d2) by means of the first joining and welding device 50, joining and welding of the second edges 52, to secure the functional objects C to the open casings E, by forming the two separate and distinct zones Z1, Z2 inside the casings E;
d3) by means of the positioning and calibrated dispensing device 10, the calibrated dispensing of the substances L near the functional objects C;
e) by means of the second joining and welding device 60, joining and welding of the third edges 61 to form the closed casings E;
f) by means of hollow cutting device 70, outer edging of the casings E; and
g) by means of the final evacuation means 80, out-feeding of the finished packages.

A first variant of the process of packaging allows carrying out almost simultaneously the following steps:
d1) joining and welding of the first edges 51;
d2) joining and sealing of the second edges 52; and
d3) calibrated dispensing of the substances L near the functional objects C.

A second variant of the process of packaging allows performing almost simultaneously by the operating unit, the following steps:
e) joining and welding the third edges 61; and
f) outer edging of the casings E.

### Industrial applicability

The system for aseptic packaging, object of the present invention allows achieving the intended aim and objects.

The main invention consists in injecting the lubricant around the catheter contained in a film protective bag by means of the same operative unit that performs the following functions: positioning of the catheter, namely the pipe and the possible jointing element, the tube previously being already glued or welded to the jointing element, in the welding mold, by means of the device for positioning and distribution; welding of the catheter to the film simultaneously to the welding of the contour of the film leaving an opening for the introduction of the lubricant; introduction of the lubricant, by means of the positioning and calibrated dispensing device; completion of the welding.

In particular, the region not affected by lubricant can indifferently be welded before or after the introduction of the lubricant and extraction of the device for positioning and calibrated distribution to allow the complete welding of the film protective bag, which prevents the leakage of the lubricant.

The catheter originates a further problem, both when it consists of a simple pipe and when it consists of a tube with a joint, formed by a plastic element with a mass greater than that of the film at which the catheter is to be welded.

Due to the above, the catheter requires for its welding to the film an energy by far greater than the energy required for the welding of the films of the peripheral region. Obtaining the welding of the junction and of the peripheral zone requires adjustments in the welding mold in order to manage different temperature zones.

In the case of thermal welding of film and catheter, the above-mentioned problem is solved by means of a mold designed and constructed in a manner suitable for managing zones at different temperatures and therefore to allow the simultaneous welding along the perimeter of the films as well as between the catheter and the film.

In detail, the simultaneous welding mold comprises a half-mold or a lower half-mold and an upper half-mold both provided with thermoregulated zones of different temperature. An insulating material can be interposed between the different zones to improve the definition of the different temperature zones.

Currently, a packaging process of lubricated catheters comprises the following steps: unwinding of the PVC upper and lower film; insertion of the catheter together with its joining element between the two films, to allow the welding of the joining element that separates the packaging in two distinct zones, approximate cycle time: four (4) seconds; distribution of the lubricant, approximate cycle time: four (4) seconds; completion of the welding, approximate cycle time: six (6) seconds; punching.

By means of a first connection and welding device, only the welding of the joining component is carried out.

By means of a second device for joining and welding, the dispensing of lubricant is carried out first, by inserting a cannula in the middle of the press mold and then, after having removed the cannula, the entire perimeter is welded.

The phases of dispensing of lubricant and welding completion are carried out in sequence in the same operating unit, therefore the production cycle time is defined by the sum of the two phases, indicative cycle time: about 10 seconds.

The packaging system object of the invention implies the dispensing of the lubricant performed in the first joining and welding device as well as performing first the welding of the joining element and of a part of the peripheral zone of the packaging. The lubricant is then injected in one of the zones which divide the packaging maintaining the measured glycerin inside of it.

The step of perimetric welding is performed by means of the second joining and welding device only. In this way, the cycle time is cut down to eight (8) seconds about.

Furthermore, the welding mold is made easier to be produced by not having to weld it in two phases and it is possible to make a shorter glycerine injection cannula for there is no need to overcome the space between the welding zone of the junction element in order to enter the second device of union and welding. Currently, the use of a long cannula causes complications.

## Claims

1. System for aseptic packaging (1), regarding casings (E) containing functional objects (C) in contact with substances (L), by positioning and finishing of the casings (E) and of the functional objects (C) and by dispensing of the substances (L), comprising means for positioning of the open casings (E), positioning and finishing of the functional objects (C) inside the open casings (E), calibrated dispensing of the substances (L) inside the open casings (E), and closing and finishing of the casings (E), **characterized in that** it comprises a positioning and calibrated dispensing device (10) which allows positioning of the functional objects (C) inside the open casings (E) and the calibrated dispensing of the substances (L) inside the open casings (E).

2. System for aseptic packaging (1), according to the preceding claim, **characterized in that** the positioning of the open casings (E) regards a first film layer (E1) superimposed and facing a second film layer (E2), to allow the positioning of the functional objects (C) between the first and the second film layer (E1), (E2) and the calibrated dispensing of the substances (L) close to the functional objects (C), through said positioning and calibrated dispensing device (10).

3. System for aseptic packaging (1) according to one of the preceding claims, **characterized in that** said positioning and calibrated dispensing device (10) comprises a robotic arm (101) connected to gripping means (102) and to intercepting means of substances (103), said gripping means (102) comprising at least a resilient tubular element (2) shaped to be joined at least at one point of the functional objects (C) to permit positioning of the functional objects (C), said intercepting means of substances (103) being connected to said at least one resilient tubular element (2), to allow the dispensing of substances (L).

4. System for aseptic packaging (1), according to one of the preceding claims, **characterized in that** it comprises in-feeding means (20) of the first film layer (E1) and of the second film layer (E2), combined with a set of transmission, retraction and accumulation / release elements to enable the first film layer (E1) to be configurated and superimposed over and facing the second film layer (E2), any printing means (30) to graphically impress at least one of the film layers (E1), (E2), in-feeding means (40) of the functional objects (C), a first joining and welding device (50) to form the open casings (E), by joining and welding of first edges (51), as well as allowing to secure the functional objects (C) to the open casings (E), by joining and welding of second edges (52), creating two separate and distinct zones (Z1), (Z2), inside the casings (E), a second joining and welding device (60) to form the closed casings (E), by joining and welding of third edges (61), a hollow cutting device (70) for the outer edging of the casings (E) and final evacuation means (80).

5. System for aseptic packaging (1) according to the preceding claim, **characterized in that** said first joining and welding device (50) for forming the open casings (E) and said second joining and welding device (60) for forming the closed casings (E) are reciprocally placed at a distance (Dsinc) in order to be able to operate simultaneously on casings (E) respectively open and closed, realizing a continuous packing cycle.

6. System for aseptic packaging (1), according to one of the preceding claims, **characterized in that** the casings (E) are formed of thin films of polyvinyl chloride and that the functional objects (C) consist of catheters lubricated by the substances (L), such as glycerin.

7. System for aseptic packaging (1) according to the preceding claim, **characterized in that** the functional objects (C) formed by lubricated catheters comprise a plastic junction element (3), positioned and welded so as to be able to hermetically separate the two zones (Z1), (Z2) inside the casings (E), in case a small tube (4) connected to said junction element (3), said junction element (3) comprising calibrated holes (5) and a mechanical stop (6) respectively, to allow union and detachment with respect to said gripping means (102), by means of said at least one resilient tubular element (2) sliding over a bracket (7).
